(19)

Europäisches Patentamt
European Patent Office
Office européen des brevets

(11) **EP 4 324 492 B1**

(12) **EUROPEAN PATENT SPECIFICATION**

(45) Date of publication and mention
of the grant of the patent:
**04.03.2026 Bulletin 2026/10**

(21) Application number: **23461636.5**

(22) Date of filing: **11.08.2023**

(51) International Patent Classification (IPC):
**A61L 27/46** (2006.01)        **A61L 27/52** (2006.01)
**A61L 27/54** (2006.01)

(52) Cooperative Patent Classification (CPC):
(C-Sets available)
**A61L 27/46; A61L 27/52; A61L 27/54;**
A61L 2300/436; A61L 2400/06; A61L 2430/02
(Cont.)

(54) **MULTIFUNCTIONAL, HYDROGEL HYBRID MATERIAL, METHOD OF ITS PREPARATION AND USE IN THE TREATMENT OF BONE LOSSES**

MULTIFUNKTIONELLES HYDROGELHYBRIDMATERIAL, VERFAHREN ZU SEINER HERSTELLUNG UND VERWENDUNG BEI DER BEHANDLUNG VON KNOCHENVERLUSTEN

MATÉRIAU HYBRIDE D'HYDROGEL MULTIFONCTIONNEL, PROCÉDÉ POUR SA PRÉPARATION ET SON UTILISATION DANS LE TRAITEMENT DE PERTES OSSEUSES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **11.08.2022 PL 44199622**

(43) Date of publication of application:
**21.02.2024 Bulletin 2024/08**

(73) Proprietor: **UNIWERSYTET JAGIELLONSKI**
**31-007 Kraków (PL)**

(72) Inventors:
• **Lewandowska-Lancucka, Joanna**
**32-010 Luborzyca (PL)**
• **Klara, Joanna**
**30-364 Kraków (PL)**

(74) Representative: **Witek, Rafal**
**WTS Patent Attorneys**
**Witek, Sniezko & Partners**
**ul. Weigla 12**
**53-114 Wroclaw (PL)**

(56) References cited:
**WO-A1-2022/045910        PL-A1- 435 104**

• **KLARA JOANNA ET AL: "Lysine-functionalized chondroitin sulfate improves the biological properties of collagen/chitosan-based injectable hydrogels", INTERNATIONAL JOURNAL OF BIOLOGICAL MACROMOLECULES, ELSEVIER BV, NL, vol. 202, 14 January 2022 (2022-01-14), pages 318 - 331, XP086973772, ISSN: 0141-8130, [retrieved on 20220114], DOI: 10.1016/ J.IJBIOMAC.2022.01.026**
• **GISBERT-GARZAR◇N MIGUEL ET AL: "Mesoporous Silica Nanoparticles for the Treatment of Complex Bone Diseases: Bone Cancer, Bone Infection and Osteoporosis", PHARMACEUTICS, vol. 12, no. 1, 20 January 2020 (2020-01-20), CH, pages 83, XP093114284, ISSN: 1999-4923, DOI: 10.3390/ pharmaceutics12010083**
• **WEI HUANG ET AL: "Alendronate decorated nano hydroxyapatite in mesoporous silica: Cytotoxicity and osteogenic properties", APPLIED SURFACE SCIENCE,, vol. 257, no. 23, 1 June 2011 (2011-06-01), pages 9757 - 9761, XP028264679, [retrieved on 20110612], DOI: 10.1016/J.APSUSC.2011.06.002**

EP 4 324 492 B1

(52) Cooperative Patent Classification (CPC): (Cont.)

    C-Sets
    **A61L 27/46, C08L 5/08;**
    **A61L 27/46, C08L 89/06**

**Description**

The invention relates to a multifunctional improved hybrid material with therapeutic potential, a method for obtaining such hydrogel materials and their use in regenerative medicine, in particular for the treatment of bone defects caused by osteoporosis.

Osteoporosis is one of the most progressive, systemic and metabolic bone diseases, characterised by a decrease in bone mass and microstructural damage to bone tissue. Due to an imbalance between the processes of bone formation and absorption, the disease increases the predisposition to osteoporotic fractures even after minor trauma and thus significantly impairs patients' quality of life. Despite the progressive breakdown of bone mass, the disease does not make itself known for a long time, and osteoporotic fractures can be the first visible symptom of an often already advanced condition. Fractures of the wrist, spine and proximal femur are considered the most common fractures associated with osteoporosis. According to the World Health Organisation (WHO), older people, especially postmenopausal women, experience a significant acceleration of bone mass loss, making osteoporosis a serious problem threatening the health of people worldwide. It is estimated that more than 23 million people in the EU are at high risk of developing osteoporosis. The disease is not only a health problem, but also a socio-economic one, as millions of osteoporotic fractures and associated rehabilitation/care cost European healthcare systems more than €56 billion per year (2019 figures). Each year in Europe, a hip or spinal fracture contributes to the death of almost a quarter of a million people [Kanis, J. A. et al. (2021). Archives of osteoporosis, 16(1), 1-82]. The contribution of osteoporosis among other diseases is increasing due to the ageing population of most developed countries, including EU countries. Therefore, early prevention, as well as the development of effective methods of diagnosis and treatment, are crucial in this regard.

The main recommendations for people struggling with osteoporosis are to change dietary habits, introduce and select appropriate physical activity and supplementation with calcium and vitamin D. The supplements are an important adjunct to the treatment of osteoporosis as they influence the effectiveness of anti-osteoporotic drugs. Two classes of drugs are used in the treatment of osteoporosis - antiresorptive drugs, which slow resorption, and anabolic drugs, which stimulate bone formation. Of the antiresorptive drugs, bisphosphonates are the most numerous group. Alendronate (ALN), a bone resorption inhibitor, is the most commonly prescribed oral drug of the aminobisphosphonate group used in the treatment of postmenopausal osteoporosis, corticosteroid-induced osteoporosis, Paget's disease and metastatic bone disease. Unfortunately, the administration of ALN is associated with serious side effects, including jawbone necrosis, gastrointestinal irritation, nausea, musculoskeletal pain and even cardiovascular problems. Also, intravenous administration of **ALN** carries the risk of nephrotoxicity, flu-like symptoms and electrolyte imbalance. Therefore, systems that allow for localised administration and thus localised action of this drug appear to be an extremely attractive solution to ensure reduced bone resorption and at the same time reduce systemic side effects throughout the therapy. Various systems with potential as carriers for alendronate have been developed; however, the effects of local delivery require further optimisation and research due to common problems with insufficient encapsulation efficiency in the system as well as insufficient control of diffusion and release of the drug.

From the application P.428993 is known a biocompatible hydrogel hybrid material and a method for obtaining thereof, suitable for use in regenerative medicine, particularly in bone tissue reconstruction. The material comprises a hydrogel which is a mixture of collagen, chitosan and hyaluronic acid cross-linked with genipin together with amine group-functionalised silica particles suspended therein, the content of the functionalised silica particles being not less than 1.9 mg/mL, preferably between 1.9 and 3.8 mg/mL of the final sol. These particles had a total surface area of $S_{BET} = 24$ m$^2$/g, which classifies them as non-porous or low porosity materials [Plumeré, N. et al. (2012). Journal of colloid and interface science, 368(1), 208-219]. The mesopores determined, with diameters of D=23 nm, are not a component of the particles themselves and were formed in the gaps between the two fractions of objects present in the material obtained (particles with diameters of 400 and 100 nm). Commercially available hyaluronic acid was used to synthesise the hybrid material (it was not subjected to any modification) and accounted for 10% by weight of the biopolymer mixture. This material did not contain alendronate in its composition and had no therapeutic properties. From the application P.435104 is known a biocompatible hydrogel hybrid material and a method for obtaining thereof, in which hydroxyapatite-decorated silica particles functionalised with amine groups were used as a carrier for alendronate. These particles were decorated with hydroxyapatite, then alendronate was attached to them and suspended in a biopolymer matrix containing: collagen, chitosan, modified hyaluronic acid in a weight ratio of 5:2:3, respectively. **In** the known material, hydroxyapatite-decorated silica particles functionalised with amine groups, which had no mesopores within their structure and their total surface area was equal to 24 m$^2$/g, were used as a carrier for alendronate.

The aim of the invention is to provide a multifunctional material containing a known active substance enhancing the bone regeneration process, such as a drug from the bisphosphonate group, for example alendronate, which, while demonstrating therapeutic anti-osteoporotic potential, at the same time allows a minimally invasive implantation and control of the release process of the active substance, furthermore has the ability to bio-integrate and support bone regeneration, and is characterised by desirable mechanical properties and biodegradability. In particular, the aim of the invention is to obtain an improved hydrogel material that provides a higher loading degree with the mentioned active substance and, at the same time, reduces uncontrolled leakage of the drug from the hydrogel matrix.

The subject of the invention is a multifunctional hydrogel hybrid material characterised in that it comprises:

a) a biopolymer matrix comprising: collagen, chitosan, lysine modified chondroitin sulphate,
b) amine-functionalised mesoporous silica-apatite particles with a total $S_{BET}$ surface area of not less than 613 $m^2$/g and mesopores (2-50 nm) formed in the particle structure with a volume of not less than 0.57 $cm^3$/g,
c) an active substance selected from the known bisphosphonate drugs, particularly alendronate, attached to the silica-apatite particles,
d) a cross-linking agent.

Preferably, the biopolymer matrix comprises: collagen, chitosan, modified chondroitin sulphate in a weight ratio of 5:2:3, respectively.

Preferably, the cross-linking agent is genipin.

A further subject of the invention is a method for obtaining the multifunctional hydrogel hybrid material characterised in that it comprises the following steps:

a) amine-functionalised mesoporous silica particles are obtained by the reaction of cetyltrimethylammonium bromide, tetraethoxysilane and aminopropyltriethoxysilane, carried out in aqueous solution in an alkaline environment, the resulting material is then separated and treated with an ammonium nitrate solution in ethanol, preferably with stirring for approximately 18 hours at 80°C, and the resulting amine-functionalised mesoporous silica particles (MSP-NH$_2$) are then isolated,
b) the MSP-NH$_2$ particles obtained in step a) are suspended in an aqueous solution of SBF, preferably at a concentration of 1.5 M, to obtain, after 5 days of incubation, mineral phase-coated silica particles (MSP-NH$_2$-HAp),
c) sodium alendronate is attached to the particles obtained in step b),
d) an aqueous suspension of particles from step c) is added to the mixture of collagen, chitosan and lysine-modified chondroitin sulphate,
e) the mixture obtained in step d) is subjected to a cross-linking reaction with genipin. Preferably, the method of the invention is carried out using the sol-gel method.

A further subject of the invention is a multifunctional hydrogel hybrid material as defined above or obtained by the method defined above for use in the treatment or prevention of bone tissue defects.

Preferably, the hybrid material for use according to the invention is characterised in that the bone defects are a result of osteoporosis.

According to the invention, "mesoporous silica particles functionalised with amine groups" is understood to be silica particles with a total $S_{BET}$ surface area of not less than 613 $m^2$/g and mesopores (2-50 nm) formed in the particle structure with a volume of not less than 0.57 $cm^3$/g. Preferably, the process of controlled decoration of such particles with hydroxyapatite does not take more than 5 days.

According to the invention, "mesoporous silica particles decorated with hydroxyapatite (MSP-NH$_2$-HAp)" are considered to be such particles which allow alendronate loading/attachment of not less than 10%.

Detailed description of the invention

The material of the invention is a novel multifunctional hybrid material obtained by suspending in a biopolymer sol (consisting of a collagen/chitosan/lysine-modified chondroitin sulphate mixture) mesoporous silica particles functionalised with amine groups and decorated with hydroxyapatite with an attached drug from the bisphosphonate group, preferably alendronate. The hybrid systems obtained consist of a selected ColChCS$_{mod}$30_20 hydrogel (50% by weight of collagen (Col), 20% by weight of chitosan (Ch) and 30% by weight of amine group-functionalised chondroitin sulphate (CS$_{mod}$) cross-linked with 20 mM genipin solution and a bioactive carrier with loaded/attached drug (MSP-NH$_2$-HAp-ALN particles), which was suspended in a polymer sol and then covalently attached to the biopolymer matrix.

The material of the invention is therefore a new multifunctional hybrid material obtained by suspending mesoporous silica particles functionalised with amine groups and decorated with hydroxyapatite with alendronate attached to its surface in a biopolymer sol (consisting of a mixture of collagen/chitosan/lysine-modified chondroitin sulphate). The mixture thus prepared is then chemically cross-linked with a substance of natural origin (genipin) to obtain a structurally stable hybrid material, in which the particles are attached by covalent bonds to the biopolymer hydrogel matrix. The chemically cross-linked system thus constructed acts as a system for controlled and localised delivery of the therapeutic substance (ALN). Furthermore, the carefully selected composition of this material provides its biointegration into natural bone (decoration of the carrier with a mineral phase), as well as providing a convenient biomatrix for colonisation by osteoblastic cells (biomimetic hydrogel matrix) and desirable mechanical properties.

An important advantage of the material described in the application is that it is in an injectable form, thus enabling application in a minimally invasive manner. This means that therapy with this material does not require conventional surgery and the administration itself can take place in a doctor's office.

A particularly beneficial application of the material described in the application is the treatment or reconstruction of small bone defects caused by osteoporosis. It can also be used to support the treatment of various types of small orthopaedic defects as well as dental defects (e.g. jawbone defects after tooth extraction), and can also be used as an alternative filling for augmentation screws used in neurosurgical procedures.

What distinguishes the presented invention from solutions known from the state of the art (in particular the material from patent application P.428993) is the use of amine group-functionalised mesoporous silica particles (MSP-$NH_2$) decorated with hydroxyapatite as an ALN carrier. The excellent textural properties of the obtained MSP-$NH_2$ including a large total surface area ($S_{BET}$ ~613 $m^2$/g; the total surface area for the $SiO_2$-$NH_2$ particles presented in P.428993 was $S_{BET}$ ~24 $m^2$/g) provided more efficient drug loading (10-14%; for the particles presented in P.428993, the loading efficiency was 3%). Furthermore, adsorption into the carrier mesopores is further enhanced by interactions between the amine groups of the matrix and the phosphate groups of the ALN while allowing covalent attachment of the MSP to the biopolymer network during the crosslinking process. This approach will eliminate uncontrolled 'leakage' of MSP particles from the implantation sites under *in vivo* conditions thus ensuring that the designed functionality of the Invention is maintained. In addition, the high density of silanol (SiOH) groups on the MSP surface contributed to a significant time reduction in the process of controlled apatite deposition (down to 5 days from 10 for $SiO_2$-$NH_2$ particles), which is extremely important from a technological point of view and potential applications. Moreover, the ALN-loaded mesoporous carrier was suspended and then covalently bound to the hydrogel matrix with a new composition. The use of amine group-functionalised chondroitin sulphate resulted in a biomaterial that supports bone formation, which is extremely important in the context of supporting bone tissue regeneration.

Based on the research carried out, the following unexpected beneficial properties of the material obtained were demonstrated:

It was shown that the use of MSP-$NH_2$ particles enables a significant time reduction in the process of controlled apatite deposition (down to 5 days from 10 for the $SiO_2$-$NH_2$ particles presented in patent application P.428993), which is extremely important from the technological point of view and potential applications.

It was confirmed that the use of amine group-functionalised mesoporous silica particles decorated with hydroxyapatite (MSP-$NH_2$-HAp) as an ALN carrier provides more efficient drug loading (10-14%) (for the invention presented in patent application P.428993 the loading rate was 3% (TG assay).

The injectability of the designed material was confirmed and the efficiency of the MSP-$NH_2$-HAp-ALN particles attachment to the polymer network was also demonstrated (enzymatic degradation assays, rheological studies).

The obtained profile of drug release from the hybrid material compared with the result of ALN release directly from MSP-$NH_2$-HAp-ALN particles clearly confirmed the effectiveness of the use of hydrogel matrices as a 'tool' limiting uncontrolled drug leakage.

The biocompatibility of the hybrid materials and their ability to induce the mineralisation process of MG-63 cells was demonstrated. Preliminary *in vitro* bioassays showed that the presence of MSP-$NH_2$-HAp-ALN particles does not reduce the biocompatibility of the hybrid materials (compared to the control material ColChCS$_{mod}$). ALP activity studies revealed the ability of the tested materials to induce osteoblast mineralisation.

The bioactivity of the hybrid materials under SBF conditions was confirmed. Biomineralization was observed for both the CoChCS$_{mod}$ matrix used and the hybrid systems already after 3 days of incubation of the material under simulated body fluid (SBF) conditions. The formation of a new mineral phase was confirmed that provides faster biointegration of the material into natural bone under *in vivo* conditions.

The developed hydrogel matrix using amine-functionalised chondroitin sulphate was shown to produce a biomaterial that promotes bone formation (biomineralization under SBF conditions was observed, as well as an increase in ALP activity of MG-63 cells for the matrix alone).

Therapeutic potential of the hybrid systems was demonstrated. Preliminary *in vitro* bioassays using J774A.1 cells showed that the designed hybrid materials have therapeutic potential manifested by impairment of the activity of model osteoclastic cells.

In summary, the proposed invention provides a non-invasive local application of the material, which increases the effectiveness of alendronate therapy while reducing the side effects associated with oral administration of the drug. The material is specifically addressed for the treatment of early osteoporotic lesions not eligible for conventional surgery. This invention is a substantially improved version of the system from patent application P.428993, with the following advantages over the known solution: (1) accelerated decoration with hydroxyapatite, (2) more efficient drug loading, (3) optimised release profile and also (4) the observed bioactivity of the new matrix with the addition of amine group-functionalised chondroitin sulphate significantly increases its application potential.

For a better understanding of the essence of the invention, the present specification is illustrated by the accompanying figures.

Fig. 1 shows (A) SEM microphotographs, (B) HRTEM images obtained for the MSP-NH$_2$ particles.

Fig. 2 summarises: (A) adsorption-desorption curves, (B) pore diameter distribution, (C) table with results of porosimetry measurements for the MSP-NH$_2$ particles.

Fig. 3 shows (A) SEM microphotographs and (B) XRD diffractogram for the MSP-NH$_2$-HAp particles.

Fig. 4 shows XPS results for the (A) MSP-NH$_2$, (B) MSP-NH$_2$-HAp and (C) MSP-NH$_2$-HAp-ALN particles.

Fig. 5 shows (A) thermogravimetric curves and (B) alendronate release curve for the MSP-NH$_2$-HAp-ALN (LC - loading capacity).

Fig. 6 shows (A) values of the elastic modulus (B) measurement result for the parameters characterising the flow point (G'=G") of the obtained hybrid systems and ColChCS$_{mod}$.

Fig. 7 shows (A) enzymatic degradation results for the obtained hybrid systems and ColChCS$_{mod}$, (B) swelling ratio for the obtained hybrid systems and ColChCS$_{mod}$.

Fig. 8 shows the wetting angle results for the hybrid systems and ColChCS$_{mod}$.

Fig. 9 shows (A) Alamar Blue test results after 1, 3 and 7 culture days of MG-63 cells on the surface of the materials, (B) alkaline phosphatase (ALP) activity test results after 3 and 7 culture days of MG-63 cells on the surface of the materials and (C) Alamar Blue test results after 1, 3 and 7 culture days of J774A.1 cells on the surface of the materials.

Fig. 10 shows SEM microphotographs for the hydrogel systems after 3.5 and 7 days of biomineralization, along with the calculated Ca/P ratio.

Fig. 11 shows a summary of ALN release profiles from the hybrid system (HybC3) and MSP-NH$_2$-HAp-ALN particles.

The following examples disclose the successive steps of an exemplary embodiment of the method of the invention and present results confirming the favourable properties of the materials obtained according to the invention.

**Example 1. Preparation and characterization of mesoporous silica particles decorated with hydroxyapatite and loaded with alendronate**

A methodology was developed to obtain hydroxyapatite-decorated mesoporous silica particles with alendronate loaded/attached (MSP-NH$_2$-HAp-ALN). The particles obtained (MSP-NH$_2$, MSP-NH$_2$-HAp and MSP-NH$_2$-HAp-ALN) were characterised in terms of morphology (SEM/HRTEM) and chemical composition (XPS, XRD, EDS, TG). Then, using UV-Vis spectrophotometric measurements, the encapsulation efficiency was determined and the kinetics of drug release under physiological conditions (into a medium with pH=7.4 and temperature = 37°C) was investigated.

The optimised methodology for obtaining mesoporous silica particles decorated with hydroxyapatite and loaded with alendronate consists of three main steps: (i) synthesis of mesoporous silica particles functionalised with amine groups (MSP-NH$_2$), (ii) hydroxyapatite decoration process under conditions simulating human plasma (MSP-NH$_2$-HAp), (iii) alendronate loading/attachment (MSP-NH$_2$-HAp-ALN).

(i) Synthesis of mesoporous silica particles functionalised with amine groups (MSP-NH$_2$)

0.2 g of cetyltrimethylammonium bromide (CTAB) was dissolved in 100 mL of distilled water and 1.4 mL of 1 M sodium base, NaOH, was added. The solution was stirred for 2 h (80°C, 500 rpm). Then 2 mL of tetraethoxysilane (TEOS) and 0.2 mL of aminopropyltriethoxysilane (APTES) were added dropwise and the reaction was continued under the same conditions for a further 2 hours. The resulting material was cooled and centrifuged (5°C, 9000 rpm, 10 minutes). The precipitate was washed twice with water and three times with 96% ethanol (EtOH) solution. 2 g of ammonium nitrate (NH$_4$NO$_3$) was dissolved in 200 ml of 96% ethanol and added to the resulting precipitateto remove residual CTAB. The resulting suspension was stirred (500 rpm) at 80°C for 18 hours. The resulting material (MSP-NH$_2$) was centrifuged and washed three times with 96% EtOH and three times with water, frozen and then freeze-dried for 24 hours.

(ii) Decoration of MSP-NH$_2$ with hydroxyapatite (MSP-NH$_2$-HAp)

In order to decorate the MSP-NH$_2$ particles with hydroxyapatite, 20 mg of MSP-NH$_2$ were suspended in 20 mL of a modified simulated body fluid(1.5x SBF), by placing in an ultrasonic bath for 5 minutes. The resulting suspension was then incubated at 37°C with gentle shaking (50 rpm). After 24 h, the material was centrifuged (5°C, 10,000 rpm, 20 min) and a fresh aliquot of 1.5x SBF was added. The incubation was carried out for 5 days. After decoration, the material was washed 3x with distilled water, frozen and freeze-dried for 24 h.

(iii) Loading of alendronate (MSP-NH$_2$-HAp-ALN)

20 mg of sodium alendronate (ALN) was dissolved in 10 mL of 5 mM NaOH and the pH of the solution was raised to 10 with 20 mM NaOH. 20 mg of MSP-NH$_2$-HAp particles were suspended in 3 mL of 5 mM NaOH solution by placing in an ultrasonic bath for 5 minutes. Alendronate solution was added to the suspension containing MSP-NH$_2$-HAp and stirred (500 rpm) at 37°C for 72 hours. After 3 days, the product (MSP-NH$_2$-HAp-ALN) was centrifuged (5°C, 10,000 rpm, 20 minutes), washed with deionised water, frozen and freeze-dried for 24 h.

Properties of the obtained particles

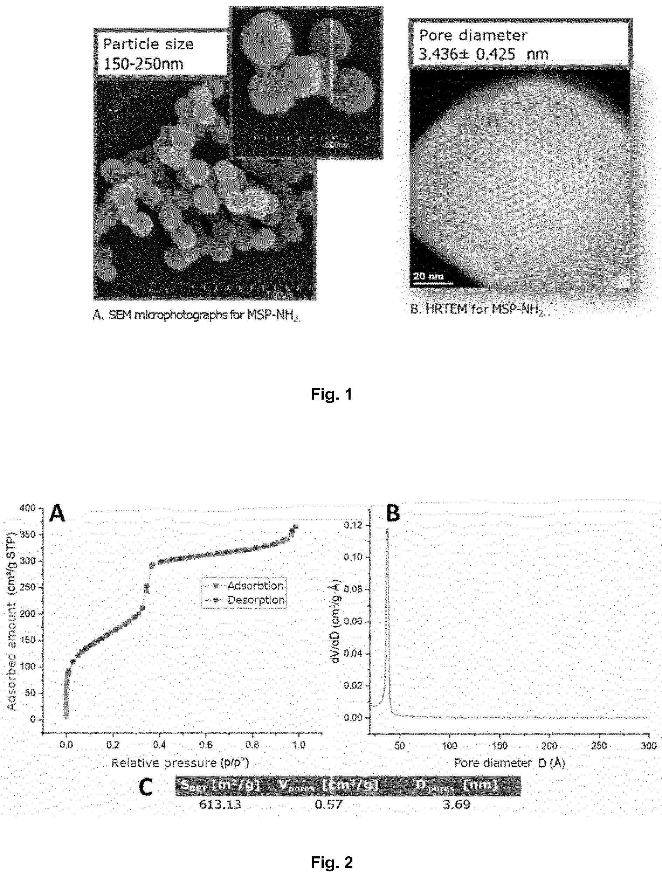

The materials obtained after the freeze-drying process were examined using a series of methods. Studies with SEM and HRTEM techniques revealed that the synthesized MSP-NH$_2$ particles exhibited a spherical morphology with diameters ranging from 150 to 250 nm and pores with a diameter (d) of 3.5 nm (**Fig. 1**).

Based on porosimetry studies, it was determined that the obtained structures had a BET surface area of 613 m$^2$/g, as deduced from the adsorption-desorption isotherms of type IVb (**Fig. 2A**), with pore volumes of 0.57 cm$^3$/g and an average pore diameter of 3.7 nm. According to the literature, the obtained pore sizes, both from porosimetry measurements and HRTEM images, fall within the range characteristic to mesopores. Additionally, for MSP-NH$_2$, these pores exhibited a narrow size distribution (**Fig. 2B**). The values obtained from porosimetry measurements are summarized in Table in **Fig. 2C**.

For comparison, silica particles functionalised with amine groups as described in the prior art were obtained without the use of a surfactant matrix. The obtained particles were characterised by a low total surface area, S$_{BET}$ = 24 m$^2$/g, classifying them as non-porous or low porosity materials [Plumeré, N. et al. (2012). Journal of colloid and interface science, 368(1), 208-219]. The determined mesopores with diameters of D = 23 nm are not inherent to the particles themselves but result from the presence of gaps formed between two fractions of objects present in the obtained material (particles with diameters of 400 and 100 nm). A comparison of porosimetric parameters for SiO$_2$-NH$_2$ and MSP-NH$_2$ particles is presented in **Table 1.**

**Table 1.** Comparison of porosimetric parameters for SiO$_2$-NH$_2$ and MSP-NH$_2$ particles

|  | S$_{BET}$ [m$^2$/g] | V$_{mes}$ [cm$^3$/g] | D$_{mes}$ [nm] |
|---|---|---|---|
| **SiO$_2$-NH$_2$** | 24 | 0.14 | 23.00 |
| **MSP-NH$_2$** | 613 | 0.57 | 3.69 |

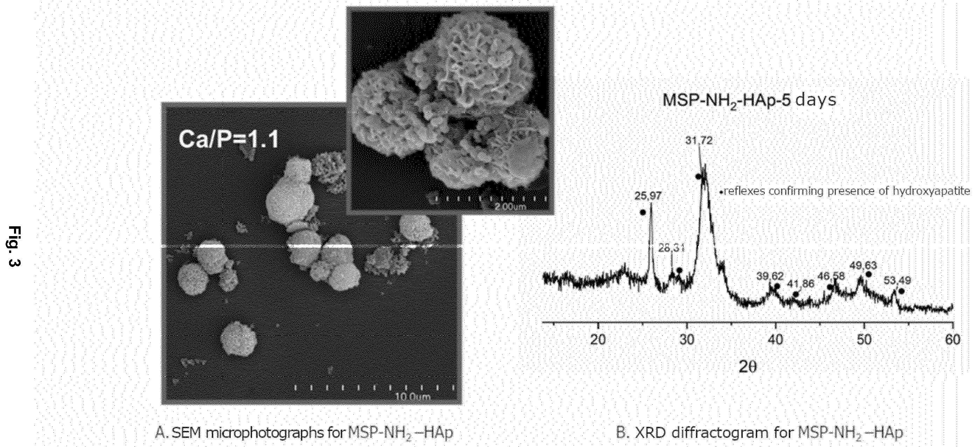

The synthesized MSP-NH$_2$ particles were subsequently subjected to hydroxyapatite decoration by incubating them in a modified simulated body fluid (1.5 SBF) at 37°C for 3, 5, 7, and 10 days. It was demonstrated that a 5-day incubation is sufficient to achieve effective hydroxyapatite deposition on the surface of MSP particles. The obtained materials were characterised using a range of complementary methods. SEM/EDS/XRD analyses confirmed the presence of hydroxyapatite on the particles after 5 days of incubation **(Fig. 3).**

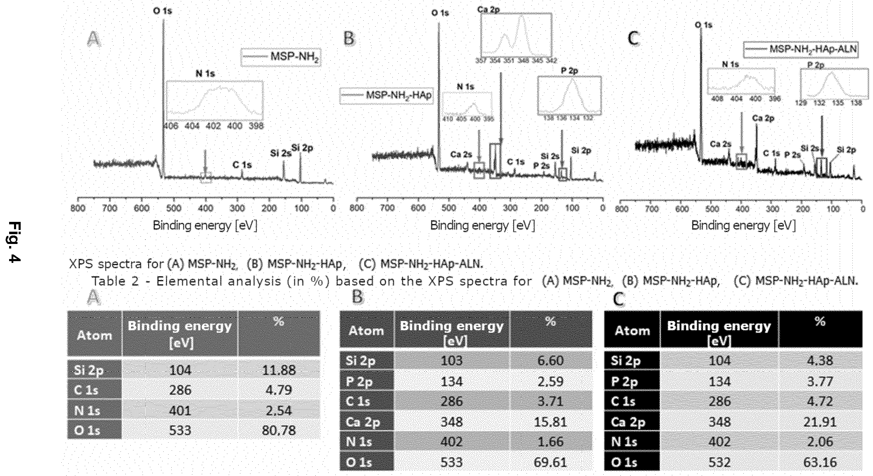

In the next step, alendronate was attached/loaded onto MSP-NH$_2$-HAp particles. The particles were incubated in an ALN solution (2 mg/mL in 5 mM NaOH, pH = 10) for 72 hours at 37°C. Subsequently, the material was centrifuged, washed with water, and isolated through freeze-drying. The obtained material (MSP-NH$_2$-HAp-ALN) was characterised for morphology (SEM) as well as chemical composition (XPS), confirming the presence of the drug in the obtained particles **(Fig. 4).**

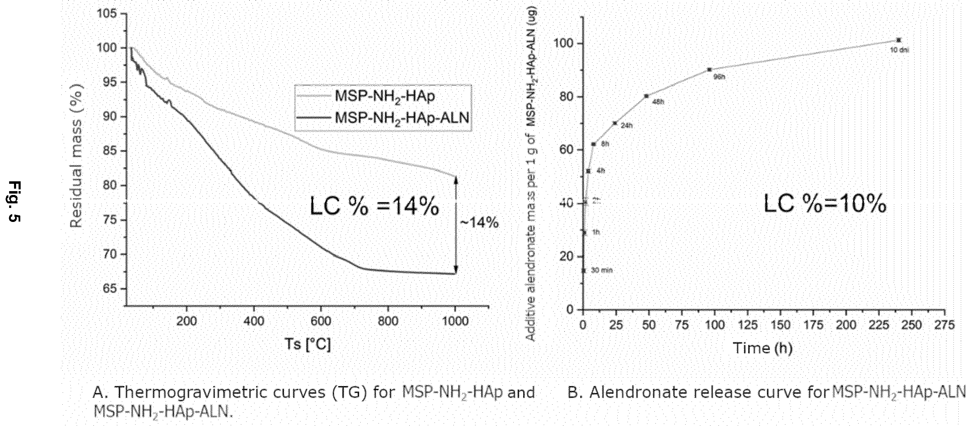

Spectrophotometric measurements and thermogravimetric analysis allowed the estimation of drug loading capacity (LC), which falls within the range of 10-14% **(Fig. 5).** After the attachment/loading of alendronate onto the obtained particles, drug release studies were also conducted under physiological conditions (pH = 7, temperature = 37°C). Measurements were performed using UV-VIS spectroscopy. The release profile, based on a 10-day experiment, indicated the occurrence of a burst effect, suggesting the need for additional 'safeguarding' of the drug.

It was thus confirmed that the use of amino-functionalised mesoporous silica particles decorated with hydroxyapatite (MSP-NH$_2$-HAp) as an ALN carrier provides more efficient drug loading (10-14%) (compared to the 3% loading reported in the patent application P.428993). Adsorption in the mesopores of the carrier is further enhanced by interactions between the amine groups of the matrix and the phosphate groups of ALN, while simultaneously allowing for the covalent attachment of MSP to the biopolymer network during the crosslinking process. This eliminates uncontrolled 'leakage' of MSP-NH$_2$ particles from implantation sites under *in vivo* conditions, thus ensuring the preservation of the desired material characteristics.

**Example 2: Preparation and physicochemical characterization of injectable hybrid systems comprising an optimized polymer matrix and mesoporous silica particles loaded with a drug and decorated with hydroxyapatite**

Preparation of the hybrid material based on a polymer matrix consisting of 30% by weight of lysine-functionalised chondroitin sulphate ($CS_{mod}$)

The procedure for obtaining lysine-modified chondroitin sulphate was described in the publication (J. Klara, A. Marczak, A. Latkiewicz, W. Horak, J. Lewandowska-Łaańcucka*, *Lysine-functionalised chondroitin sulfate improves the biological properties of collagen/chitosan-based injectable hydrogels,* Int. J. Biol. Macromol, 202, 318-331, 2022, https://doi.org/10.1016/j.ijbiomac.2022.01.026).

The synthesis was carried out in MES buffer, which was prepared by dissolving 0.97 g of 2-(N-morpholino) ethanesulfonic acid (MES) in 100 mL of deionized water and adjusting the pH to 4 using a 0.1 M NaOH solution. 500 mg of chondroitin sulphate (CS) was dissolved in 20 mL of MES buffer (50 mM, pH=4) and left on a magnetic stirrer for 24 h. Next, 730 mg of lysine, 360 mg of EDC (N-(3-dimethylaminopropyl)-N'-ethylcarbodiimide hydrochloride), and 220 mg of NHS (N-hydroxysuccinimide) were added sequentially (each of the reagents was first dissolved in 5 mL of MES buffer). The mixture was stirred with a magnetic stirrer for 24 h at room temperature. Purification of the product involved overnight dialysis against a 0.1 M aqueous solution of $Na_2CO_3$ (lasting approximately 18 hours), followed by two weeks of dialysis against water. The resulting mixture was concentrated by evaporation, frozen, and freeze-dried. The degree of lysine substitution in the isolated product ($CS_{mod}$) was determined using elemental analysis and $^1$H NMR spectroscopy, and it was found to be approximately 21%.

Using the lysine-functionalised chondroitin sulphate ($CS_{mod}$), a series of hybrid systems were created, composed of the hydrogel ColChCS$_{mod}$30_20 (a system consisting of 50% by weight collagen (Col), 20% by weight chitosan (Ch), and 30% by weight lysine-functionalised chondroitin sulphate ($CS_{mod}$), cross-linked with a 20 mM genipin solution) and a bioactive carrier loaded/attached with a drug (MSP-NH$_2$-HAp-ALN particles), which were suspended in the polymer gel and covalently attached to the biopolymer matrix. To achieve this, 520 µL of collagen solution (Col) (a solution of 4.06 mg/mL in hydrochloric acid - provided by the manufacturer, BD Biosciences) was mixed with 84 µL of chitosan solution (Ch) (1% by weight solution in 1% acetic acid), 126 µl of lysine-modified chondroitin sulphate solution ($CS_{mod}$) (1% by weight solution in 10x phosphate-buffered saline (PBS)), and 100 µl of an aqueous suspension of MSP-NH$_2$-HAp-ALN particles obtained in **Example 1** (with weights of 2.5 mg, 1.25 mg, and 0.5 mg, respectively, each suspended in 100 µL of water). The resulting sol was vigorously shaken, and then 170 µL of genipin solution (20 mM solution prepared in 10x PBS) was added and incubated at 37°C until complete crosslinking occurred. The resulting material takes the form of a hydrogel. The weight ratio of biopolymers in the obtained material was Col:Ch:$CS_{mod}$ - 5:2:3.

Using the described method, three types of hybrid systems were obtained, consisting of the hydrogel ColChCS$_{mod}$30_20 and MSP-NH$_2$-HAp-ALN particles at concentrations in the sol of 0.5, 1.25, and 2.5 mg/mL of sol, respectively. The obtained systems were designated as Hyb_C1 (for 0.5 mg/mL), Hyb_C2 (for 1.25 mg/mL), and Hyb_C3 (for 2.5 mg/mL). A control material without the addition of particles was labelled as ColChCS$_{mod}$. Three contents of MSP-NH$_2$-HAp-ALN particles (0.5, 1.25 and 2.5 mg/mL of sol) and their impact on the physicochemical properties of the obtained hybrid materials were tested. Physical and chemical studies were conducted to evaluate the efficiency of carrier attachment to the polymer network (enzymatic degradation studies, swelling, rheological studies). Wettability properties and drug release kinetics from the hydrogel system under physiological conditions were also investigated.

***Rheological studies***

Rheological studies were conducted on a series of gelled hybrid materials and a control sample (ColChCS$_{mod}$) using a rotational rheometer MCR 301 (Anton Paar) equipped with a P-PTD200 measurement cell and an H-PTD200 chamber, providing precise temperature control during measurements. Measurements were performed using a plate-plate measurement geometry with a plate diameter (d) of 25 mm and a gap height (h) of 0.1 mm, at a temperature (t) of 37°C. After placing the material on the lower measurement plate of the rheometer, excess water was drained. Subsequently, the upper rheometer plate was lowered according to an optimized scheme: 2; 1.5; 1; 0.8; 0.6; 0.5; 0.1 mm. Amplitude sweep (AS) tests were carried out at a constant frequency (f = 1 Hz) with amplitudes ranging from $\gamma$ = 0.1-1000%. At least three independent experiments were conducted for each material.

The storage modulus (G') and loss modulus (G") curves were recorded as functions of strain ($\gamma$) in the range of 0.1-1000%. According to literature data, when G" > G', the sample behaves like a viscous liquid, whereas when G' > G", the material exhibits properties of an elastic solid body. For all tested samples, higher values of the storage modulus were observed compared to the loss modulus, indicating that the obtained hybrid materials as well as the ColChCS$_{mod}$ hydrogel exhibit a predominance of elastic properties over viscous properties, which is a result of effective cross-linking. The amplitude sweep experiment also allowed the assessment of the stability of the studied systems at a constant frequency and variable strain amplitude and determination of the linear viscoelastic range (LVE). Measurements carried out within the LVE ensure that the measured hydrogel properties are independent of the applied deformation amount. It was shown that for most samples, this range extends up to $\gamma \leq 5\%$, so the analysis of the data was based on values determined at $\gamma$ = 1%.

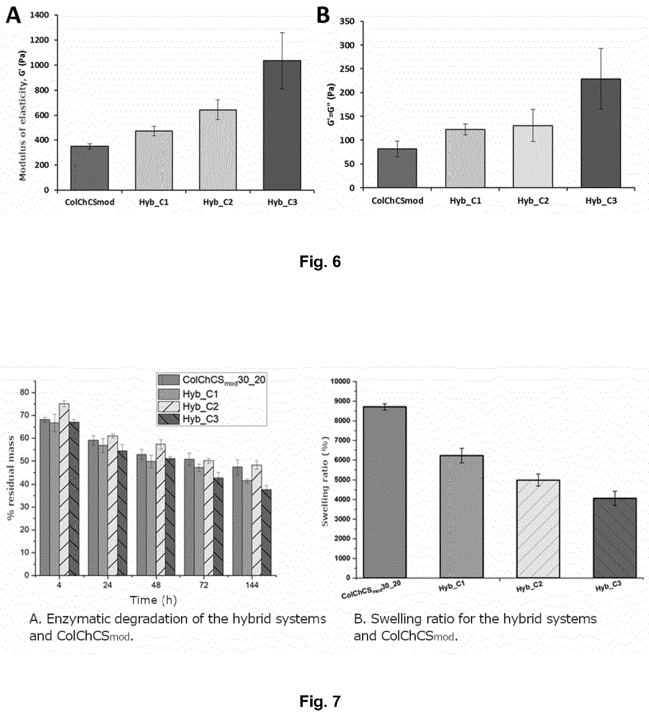

**Fig. 6A** presents the values of the storage modulus (G') obtained for the tested materials. G' is a parameter describing the elastic behaviour of the sample, and its value increases with the degree of material cross-linking. Therefore, the higher the G' value, the stiffer the system is, indicating better mechanical properties. Based on the rheological studies, it can be concluded that the introduction of MSP-NH$_2$-HAp-ALN particles into the polymer matrix significantly contributes to the improvement of mechanical properties. The higher the particle concentration, the higher the modulus of elasticity value. For the ColChCS$_{mod}$ matrix, G' = 350 Pa, while for the system with the highest particle content (Hyb_C3), this value is almost three times higher (G' = 1035 Pa). **Fig. 6B** shows the determined values of the intersection of the storage modulus (G') and loss modulus (G") curves (G' = G") characterising the flow point. Here again, a clear trend can be identified: as the particle content increases, the value of the intersection point takes higher values. This means that the hybrid materials exhibit significantly higher average yield stress compared to the control sample (ColChCS$_{mod}$), indicating greater resistance to mechanical damage. Hence, the results of rheological tests confirm the achievement of structurally stable hybrid materials.

### Degradation studies

Degradation studies **(Fig. 7A)** were conducted in a medium based on collagenase solution at a concentration of 0.2 mg/mL in PBS containing 0.36 mM CaCl$_2$. At specified time points, the solution was collected, and the materials were weighed before being treated with fresh medium. All tested materials exhibited gradual degradation throughout the entire period studied. The results confirm that the introduction of inorganic particles into hydrogel matrices does not reduce their stability and induce disintegration. After the experiment (144 hours), Hyb_C2 showed the highest resistance to degradation, comparable to the system without particles. This demonstrates the absence of 'leakage' of particles from the obtained hybrid materials, indirectly indicating the effective attachment of MSP-NH$_2$-HAp-ALN to the polymer matrix during the cross-linking process with genipin.

### Swelling studies

Swelling experiments **(Fig. 7B)** were conducted by incubating the hybrid systems in a PBS solution for 24 hours, after which the materials were weighed, freeze-dried, and weighed again. The swelling ratio (SR) was calculated using the formula: $SR = \frac{W_s - W_d}{W_d} \cdot 100\%$, where Ws is the mass before freeze-drying, and Wd is the mass after freeze-drying. The obtained hybrids are capable of swelling, with the SR values ranging from 4062 to 6225 **(Fig. 7B).** It was confirmed that the addition of MSP-NH$_2$-HAp-ALN reduces the swelling of the formed systems compared to the hydrogel matrix (8711). Furthermore, it was observed that this effect is more pronounced with increasing particle concentration in the system.

### Wettability studies

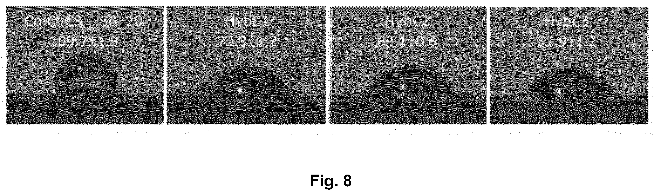

Wettability studies **(Fig. 8)** revealed that the hydrogel sample (ColChCS$_{mod}$30_20) exhibited low wettability (wetting angle of 109.7±1.9). A significant decrease in this parameter was observed when even low concentrations of particles were used in the hybrid system (72.3±1.2 for Hyb_C1). With increasing particle concentration, the wetting angle decreased, making the hybrids more hydrophilic, with the lowest value of 61.9±1.2 measured for Hyb_C3. This observation can be explained by the presence of a large number of hydroxyl groups (OH) in the particles, derived from hydroxyapatite, alendronate, and amine-functionalised mesoporous silica particles, as well as other hydrophilic groups. However, the change in wetting angle values between systems with different particle concentrations was less pronounced compared to the control hydrogel sample. The hydrophilicity of materials prepared for bone tissue engineering affects their suitability and ability to effectively adhere to cells and promote cell proliferation. Increased surface wettability of Ti, for example, has been shown to improve osteointegration in the presence of osteoporosis [Siqueira, R. et al. (2021). J. Periodontal Res. 56, 351-362] and enhance bone formation through the regulation of angiogenesis, bone mineralization, and remodelling [Calciolari, E. et al. (2018). J. Periodontal Res. 53, 598-609, Boyan, B. D. et al. (2017). Tissue Eng. - Part A 23, 1479-1489].

### Example 3. *In vitro* biological studies of selected hybrid systems

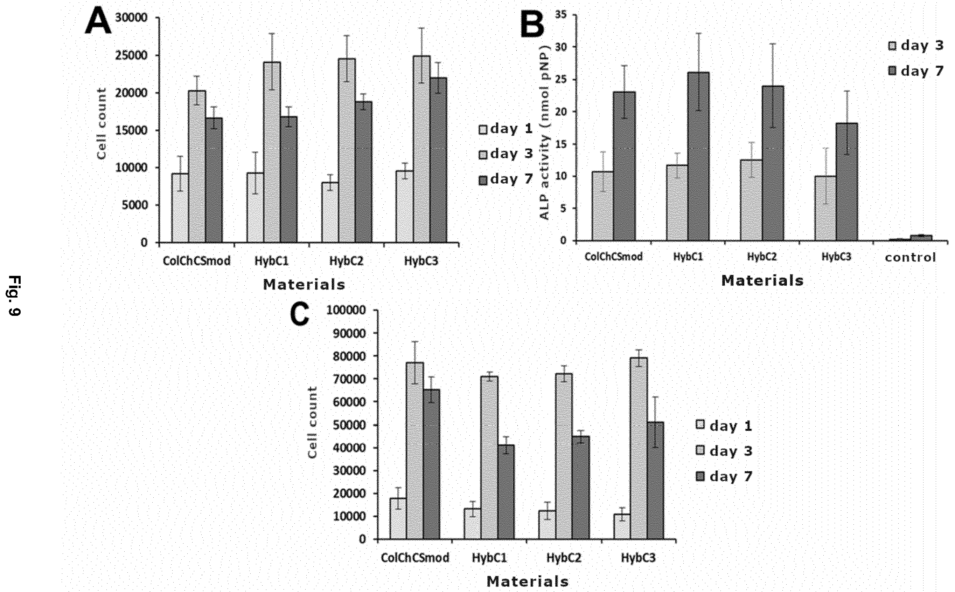

The obtained hybrid materials were subjected to preliminary *in vitro* biological studies using MG-63 osteoblastic cells. Cell proliferation and alkaline phosphatase activity of cells cultured on the surface of the tested materials were assessed. Analysing the results of the Alamar Blue assay **(Fig. 9A)** conducted on days 1 and 3 of culture, it can be concluded that the introduction of MSP-NH$_2$-HAp-ALN particles at the tested concentrations C1, C2, and C3 into the hydrogel matrix does not reduce the biocompatibility of the hybrid materials compared to the control system ColChCS$_{mod}$. All tested materials supported cell proliferation ability within a 3-day culture period. After 3 days of the experiment, a comparable (no statistically significant differences) increase in the number of cells on the hybrid materials was observed relative to the control. However, after 7 days of culture, a clear inhibition of cell proliferation was observed in all tested systems, which can be explained by the entry of cells into the mineralization phase. To verify this hypothesis, activity of alkaline phosphatase (ALP), a marker confirming the phenotype and mineralization of osteoblasts, was examined. The studies were conducted on days 3 and 7 of culture, and the results are presented in **Fig. 9B.** The ALP level of cells cultured on the material surfaces, both on the third and seventh days of the *in vitro* experiment, was significantly higher compared to the ALP activity of cells in the culture plate. What is most important is that all materials showed a significant increase in ALP activity after 7 days of the experiment, despite inhibited cell proliferation. This result confirms that the materials have the ability to induce osteoblast mineralization. Analysing the impact of MSP-NH$_2$-HAp-ALN particle concentration on ALP activity after 3 days of culture, no statistically significant differences were observed. On the seventh day of the experiment, cells cultured on the Hyb_C1 material showed the highest ALP activity.

To demonstrate therapeutic properties related to the materials' ability to inhibit bone resorption, preliminary *in vitro* biological studies were conducted using a model osteoclast cell line (J774A.1 cells). This cell line was selected as it is a reference line used in *in vitro* analyses of bisphosphonate compound metabolism. Proliferation tests (Alamar Blue assay) conducted after 1, 3, and 7 days of culture are presented in **Fig. 9C.** The data clearly confirm that the proliferation of osteoclastic cells cultured on the hybrid materials is inhibited after 7 days of the experiment. This effect is pronounced only in the case of hybrid materials (statistical significance when comparing results after 3 and 7 days of culture). At the same time, no significant influence of the analysed particle concentrations in the hydrogel matrix on this effect was observed. In summary, it has been demonstrated that the obtained hybrid systems with the tested content of MSP-NH$_2$-HAp-ALN (C1, C2, and C3) have therapeutic potential, resulting in the impairment of osteoclastic cell activity.

## Example 4. *In vitro* biomineralization studies under SBF conditions

In order to demonstrate that the obtained innovation will promote the integration of the material with bone and thus support the disrupted bone mineralization process in osteoporosis, its bioactive properties were examined. *In vitro* biomineralization experiments were conducted in simulated body fluid (SBF) conditions. The experiment involved incubating the materials in SBF at 37°C. Subsequently, to verify the creation of a new mineral phase at specific time points (after 3, 5, 7 days of incubation in SBF), the materials were subjected to SEM and EDS analysis.

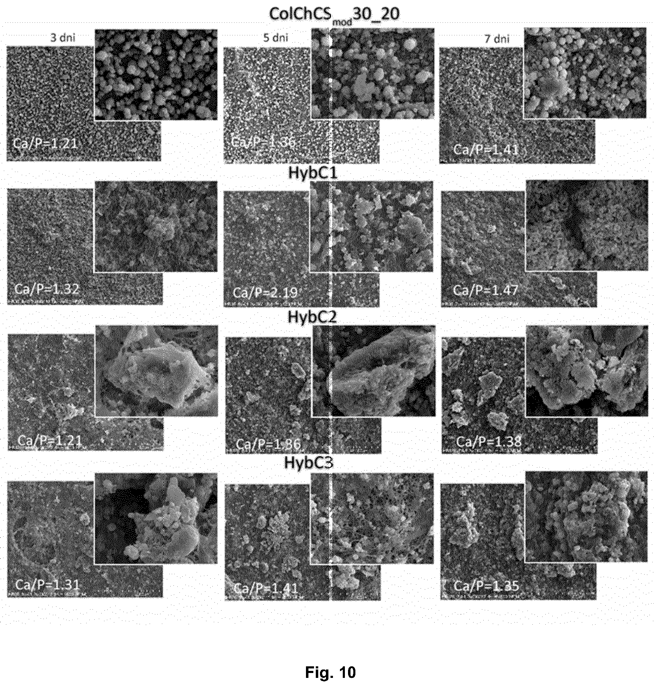

The biomineralization experiment included incubation of the obtained hybrid materials and the control material in SBF at 37°C for 3, 5, and 7 days, respectively. After this time, the materials were washed three times with water and dried. To verify the creation of a new mineral phase, the materials were subjected to SEM and EDS analysis. **Fig. 10** presents a comparison of SEM microphotographs and Ca/P ratio estimated based on the EDS analysis. It was observed that for all tested materials, both the hybrids and the hydrogel material, the creation of a new mineral phase occurred as soon as after 3 days of incubation! This is a very interesting result, clearly indicating that the ColChCS$_{mod}$30_20 matrix itself has the ability to induce mineralization. It can be noticed that in the case of the hydrogel system, both the range of the mineral phase and the Ca/P ratio increase with prolonged incubation time in SBF. In the case of hybrid systems, the extended incubation time is not directly related to the Ca/P ratio obtained; however, it was observed that higher particle concentrations in the systems promoted formation of larger aggregates of the mineral phase.

## Example 5. Effectiveness of using hydrogel matrices as a 'tool' to reduce uncontrolled drug leakage

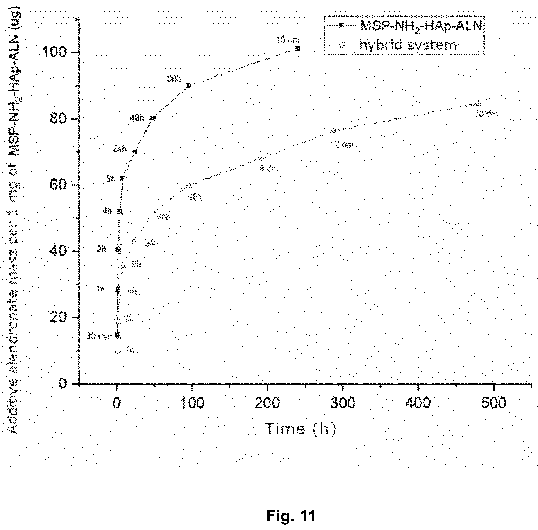

The kinetics of drug release from the hydrogel system in physiological conditions were also examined. For this purpose, the obtained hybrid system was incubated in PBS solution for 20 days (37°C, 50 rpm). At specific time intervals (1h, 2h, 4h, 8h, 24h, 48h, 96h, 192h, 288h, 480h), the solution was collected and ALN concentration determined spectrophotometrically, while the hybrids were immersed in fresh PBS. **Fig. 11** presents the drug release profile from the obtained hybrids expressed as a percentage of the total drug content in the particles. The obtained profile for the hybrid material, in comparison with the release of ALN directly from MSP-NH$_2$-HAp-ALN particles, clearly indicates the effectiveness of using hydrogel matrices as a 'tool' to limit uncontrolled drug leakage. In addition to effectively inhibiting the so-called 'burst release', extended alendronate release over time can also be observed.

## Example 6. Therapeutic effect of the obtained materials

Preliminary *in vitro* biological studies using J774A.1 cells (a model for osteoclasts) demonstrated that the material according to the invention has therapeutic potential, resulting in a significant impairment of the activity of model osteoclastic cells. This effect is significantly more pronounced compared to the material from patent application No. P.435104.

It was confirmed that the introduction of chondroitin sulphate modified with lysine into the system allowed for the creation of a biomaterial that supports the bone formation process, which is crucial for supporting bone tissue regeneration. Biomineralization was observed for both the CoChCS$_{mod}$ matrix and the hybrid systems after just 3 days of material incubation in conditions simulating plasma (SBF). The creation of a new mineral phase was confirmed, which ensures more efficient integration of the material with natural bone under *in vivo* conditions. SEM microphotographs showed that the mineralization covered large areas of the hydrogel. In the case of matrices used in the prior art (ColChHA$_{mod}$ matrix), preliminary formation of a new mineral phase with a small surface coverage of the material was observed after 3 days of incubation in SBF.

## Claims

1.  A multifunctional, hydrogel hybrid material, **characterised in that** it comprises:

    a) a biopolymer matrix comprising: collagen, chitosan, lysine modified chondroitin sulphate,
    b) amine-functionalised mesoporous silica-apatite particles with a total S$_{BET}$ surface area of not less than 613 m$^2$/g and mesopores (2-50 nm) formed in the particle structure with a volume of not less than 0.57 cm$^3$/g,
    c) an active substance selected from the known bisphosphonate drugs, particularly alendronate, attached to the silica-apatite particles,
    d) a cross-linking agent.

2.  The hybrid material of claim 1, **characterised in that** the biopolymer matrix comprises: collagen, chitosan, modified chondroitin sulphate in a weight ratio of 5:2:3, respectively.

3.  The hybrid material of claim 1, **characterised in that** the cross-linking agent is genipin.

4.  A method for obtaining a multifunctional, hydrogel hybrid material, **characterised in that** it comprises the following steps:

    a) amine-functionalised mesoporous silica particles are obtained by the reaction of cetyltrimethylammonium bromide, tetraethoxysilane and aminopropyltriethoxysilane, carried out in aqueous solution in an alkaline environment, the resulting material is then separated and treated with an ammonium nitrate solution in ethanol, preferably with stirring for approximately 18 hours at 80°C, and the resulting amine-functionalised mesoporous silica particles (MSP-NH$_2$) are then isolated,
    b) the MSP-NH$_2$ particles obtained in step a) are suspended in an aqueous solution of SBF, preferably at a concentration of 1.5 M, to obtain, after 5 days of incubation, mineral phase-coated silica particles (MSP-NH$_2$-HAp),
    c) sodium alendronate is attached to the particles obtained in step b),
    d) an aqueous suspension of particles from step c) is added to the mixture of collagen, chitosan and lysine-modified chondroitin sulphate,
    e) the mixture obtained in step d) is subjected to a cross-linking reaction with genipin.

5.  The method of claim 4, **characterised in that** it is carried out using the sol-gel method.

6.  A multifunctional, hydrogel hybrid material as defined in any of claims 1-3 or obtained by the method as defined in any of claims 4-5 for use in the treatment or prevention of bone tissue defects.

7.  The hybrid material for use of claim 6, **characterised in that** bone defects are a result of osteoporosis.

## Patentansprüche

1.  Multifunktionales Hydrogel-Hybridmaterial, **dadurch gekennzeichnet, dass** es Folgendes umfasst:

    a) eine Biopolymermatrix, umfassend: Kollagen, Chitosan, lysinmodifiziertes Chondroitinsulfat,
    b) Amin-funktionalisierte, mesoporöse Siliciumdioxid-Apatit-Partikel mit einer Gesamt-SBET-Oberfläche von mindestens 613 m$^2$/g und in der Partikelstruktur gebildeten Mesoporen (2-50 nm) mit einem Volumen von mindestens 0,57 cm$^3$/g,
    c) einen Wirkstoff, der aus den bekannten Bisphosphonat-Arzneimitteln, insbesondere Alendronat, ausgewählt ist und an die Siliciumdioxid-Apatit-Partikel gebunden ist,
    d) ein Vernetzungsmittel.

**2.** Hybridmaterial nach Anspruch 1, **dadurch gekennzeichnet, dass** die Biopolymermatrix Folgendes umfasst: Kollagen, Chitosan, modifiziertes Chondroitinsulfat in einem Gewichtsverhältnis von jeweils 5:2:3.

**3.** Hybridmaterial nach Anspruch 1, **dadurch gekennzeichnet, dass** das Vernetzungsmittel Genipin ist.

**4.** Verfahren zum Erhalten eines multifunktionalen Hydrogel-Hybridmaterials, **dadurch gekennzeichnet, dass** es die folgenden Schritte umfasst:

a) amin-funktionalisierte, mesoporöse Siliciumdioxidpartikel werden durch die Reaktion von Cetyltrimethylammoniumbromid, Tetraethoxysilan und Aminopropyltriethoxysilan in einer wässrigen Lösung in alkalischer Umgebung erhalten, das resultierende Material wird dann abgetrennt und mit einer Ammoniumnitratlösung in Ethanol behandelt, vorzugsweise unter Rühren für etwa 18 Stunden bei 80 °C, und die resultierenden amin-funktionalisierten, mesoporösen Siliciumdioxidpartikel (MSP-NH$_2$) werden dann isoliert,
b) die in Schritt a) erhaltenen MSP-NH$_2$-Partikel werden in einer wässrigen SBF-Lösung, vorzugsweise in einer Konzentration von 1,5 M, suspendiert, um nach 5 Tagen Inkubation mineralphasenbeschichtete Siliciumdioxidpartikel (MSP-NH$_2$-HAp) zu erhalten,
c) Natriumalendronat wird an die in Schritt b) erhaltenen Partikel gebunden,
d) eine wässrige Suspension von Partikeln aus Schritt c) wird zu der Mischung aus Kollagen, Chitosan und lysinmodifiziertem Chondroitinsulfat hinzugefügt,
e) die in Schritt d) erhaltene Mischung wird einer Vernetzungsreaktion mit Genipin unterzogen.

**5.** Verfahren nach Anspruch 4, **dadurch gekennzeichnet, dass** es unter Verwendung des Sol-Gel-Verfahrens durchgeführt wird.

**6.** Multifunktionales Hydrogel-Hybridmaterial nach einem der Ansprüche 1 bis 3 oder erhalten durch das Verfahren nach einem der Ansprüche 4 bis 5 zur Verwendung bei der Behandlung oder Vorbeugung von Knochengewebedefekten.

**7.** Hybridmaterial zur Verwendung nach Anspruch 6, **dadurch gekennzeichnet, dass** die Knochendefekte eine Folge von Osteoporose sind.

**Revendications**

**1.** Matériau hybride multifonctionnel à base d'hydrogel, **caractérisé en ce qu'**il comprend :

a) une matrice de biopolymère comprenant : du collagène, du chitosane, de la chondroïtine-sulfate modifiée par la lysine,
b) des particules mésoporeuses de silice-apatite à fonction amine ayant une surface spécifique totale SBET de pas moins de 613 m$^2$/g et des mésopores (2 à 50 nm) formés dans la structure particulaire d'un volume de pas moins de 0,57 cm$^3$/g,
c) une substance active sélectionnée parmi les médicaments bisphosphonate connus, en particulier l'alendronate, fixée aux particules de silice-apatite,
d) un agent de réticulation.

**2.** Matériau hybride selon la revendication 1, **caractérisé en ce que** la matrice de biopolymère comprend : du collagène, du chitosane, de la chondroïtine-sulfate modifiée dans un rapport pondéral respectivement de 5:2:3.

**3.** Matériau hybride selon la revendication 1, **caractérisé en ce que** l'agent de réticulation est la génipine.

**4.** Procédé d'obtention d'un matériau hybride multifonctionnel à base d'hydrogel, **caractérisé en ce qu'**il comprend les étapes suivantes :

a) des particules mésoporeuses de silice à fonction amine sont obtenues par la réaction du bromure de cétyltriméthylammonium, du tétraéthoxysilane et de l'aminopropyltriéthoxysilane réalisée en solution aqueuse dans un environnement alcalin, le matériau résultant est ensuite séparé et traité avec une solution de nitrate d'ammonium dans l'éthanol, de préférence sous agitation pendant environ 18 heures à 80 °C, et les particules mésoporeuses de silice à fonction amine (MSP-NH$_2$) résultantes sont ensuite isolées,
b) les particules de MSP-NH$_2$ obtenues à l'étape a) sont mises en suspension dans une solution aqueuse de SBF, de préférence à une concentration de 1,5 M, pour obtenir, après 5 jours d'incubation, des particules de silice enrobées d'une phase minérale (MSP-NH$_2$-HAp),

c) de l'alendronate de sodium est fixé aux particules obtenues à l'étape b),

d) une suspension aqueuse de particules de l'étape c) est ajoutée au mélange de collagène, de chitosane et de chondroïtine-sulfate modifiée par la lysine,

e) le mélange obtenu à l'étape d) est soumis à une réaction de réticulation avec de la génipine.

5. Procédé selon la revendication 4, **caractérisé en ce qu'**il est réalisé selon le procédé sol-gel.

6. Matériau hybride multifonctionnel à base d'hydrogel tel que défini dans l'une quelconque des revendications 1 à 3 ou obtenu par le procédé tel que défini dans l'une quelconque des revendications 4 à 5 pour une utilisation dans le traitement ou la prévention des défauts des tissus osseux.

7. Matériau hybride pour une utilisation selon la revendication 6, **caractérisé en ce que** les défauts osseux résultent d'une ostéoporose.

A. SEM microphotographs for MSP-NH$_2$.    B. HRTEM for MSP-NH$_2$.

**Fig. 1**

**Fig. 2**

B. XRD diffractogram for MSP-NH₂–HAp

A. SEM microphotographs for MSP-NH₂–HAp

**Fig. 3**

XPS spectra for (A) MSP-NH$_2$, (B) MSP-NH$_2$-HAp, (C) MSP-NH$_2$-HAp-ALN.

Table 2 - Elemental analysis (in %) based on the XPS spectra for (A) MSP-NH$_2$, (B) MSP-NH$_2$-HAp, (C) MSP-NH$_2$-HAp-ALN.

A

| Atom | Binding energy [eV] | % |
|---|---|---|
| Si 2p | 104 | 11.88 |
| C 1s | 286 | 4.79 |
| N 1s | 401 | 2.54 |
| O 1s | 533 | 80.78 |

B

| Atom | Binding energy [eV] | % |
|---|---|---|
| Si 2p | 103 | 6.60 |
| P 2p | 134 | 2.59 |
| C 1s | 286 | 3.71 |
| Ca 2p | 348 | 15.81 |
| N 1s | 402 | 1.66 |
| O 1s | 533 | 69.61 |

C

| Atom | Binding energy [eV] | % |
|---|---|---|
| Si 2p | 104 | 4.38 |
| P 2p | 134 | 3.77 |
| C 1s | 286 | 4.72 |
| Ca 2p | 348 | 21.91 |
| N 1s | 402 | 2.06 |
| O 1s | 532 | 63.16 |

**Fig. 4**

16

A. Thermogravimetric curves (TG) for MSP-NH₂-HAp and    B. Alendronate release curve for MSP-NH₂-HAp-ALN
MSP-NH₂-HAp-ALN.

**Fig. 5**

**Fig. 6**

A. Enzymatic degradation of the hybrid systems and ColChCSmod.

B. Swelling ratio for the hybrid systems and ColChCSmod.

**Fig. 7**

**Fig. 8**

Fig. 9

**Fig. 10**

**Fig. 11**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KANIS, J. A. et al.** *Archives of osteoporosis*, 2021, vol. 16 (1), 1-82
- **PLUMERÉ, N. et al.** *Journal of colloid and interface science*, 2012, vol. 368 (1), 208-219
- **SIQUEIRA, R. et al.** *J. Periodontal Res.*, 2021, vol. 56, 351-362
- **CALCIOLARI, E. et al.** *J. Periodontal Res.*, 2018, vol. 53, 598-609
- **BOYAN, B. D. et al.** *Tissue Eng. - Part A*, 2017, vol. 23, 1479-1489